# EUROPÄISCHE PATENTANMELDUNG

(11) **EP 2 829 546 A2**
(43) Veröffentlichungstag der Anmeldung: **28.01.2015**
(21) Anmeldenummer: 13198274.6
(22) Anmeldetag: 19.12.2013
(51) Int. Cl.: C07F 9/6571, C07F 9/6584, C07F 9/22

(54) **Isomerisierungsarme Hydroformylierung von Ölsäureester-haltigen Gemischen**

(30) Priorität: 23.07.2013 DE 102013214382; 23.07.2013 DE 102013214373
(71) Anmelder: Evonik Industries AG, 45128 Essen (DE)
(72) Erfinder: Dyballa, Dr., Katrin Marie,, 45657 Recklinghausen, (DE); Franke, Prof. Dr., Robert,, 45772 Marl, (DE); Börner, Prof. Dr., Armin,, 18059 Rostock, (DE); Lühr, Susan,, San Miguel, Santiago, (CL); Vilches, Dr., Marcelo,, San Miguel, Santiago, (CL)

(57) **Zusammenfassung**

Die Erfindung betrifft neue Liganden auf Basis von Derivaten der Phosphorigsäure, Zusammensetzungen, welche diese neuen Liganden aufweisen und ihre Verwendung in einem Verfahren zur isomerisierungsarmen Hydroformylierung von Ölsäureesterhaltigen Gemischen.

## Beschreibung

Die Erfindung betrifft neue Liganden auf Basis von Derivaten der Phosphorigsäure, Zusammensetzungen, welche diese neuen Liganden aufweisen und ihre Verwendung in einem Verfahren zur isomerisierungsarmen Hydroformylierung von Ölsäureester-haltigen Gemischen.

Frankel und Mitarbeiter berichteten 1969 über die Hydroformylierung von Ölsäuremethylester, der aus pflanzlichen Fettsäureestern wie Sojaöl, Leinöl und Safloröl (Diestelöl) erhalten wird. Bei relativ drastischen Bedingungen, 13,7 MPa (CO : H₂ = 1:1), 110°C, 5% Rh auf CaCO₃ und 4,4% Triphenylphosphin als Ligand, wurde als Hauptprodukt die Mischung 9/10-Formylstearate in 99% Ausbeute gebildet, wie in J. Am. Oil Chem. Soc. 1969, 46, 133 - 138 und an gleichem Ort 1971, 48, 248 - 253 offenbart wird. Nachteilig ist, dass hierbei sehr drastische Reaktionsbedingungen, wie z.B.Drücke > 10 MPa, notwendig waren.

Einige Jahre später berichtete Friedrich über die Hydroformylierung von Ölsäuremethylester bei wesentlich niedrigeren Drücken von 1,38 - 6,21 MPa mit Triphenylphosphit bzw. Triphenylphosphin als Liganden, wie in Ind. Eng. Chem. Res. Dev. 1978, 17, 205 - 207 offenbart wird. Der Aldehyd wurde mit bis zu 97% Ausbeute gebildet. Mit Triphenylphosphit nahm die Regioselektivität zur Bildung des 9/10-Formylprodukts zu. Nachteilig ist, dass hierbei sehr drastische Reaktionsbedingungen, wie z.B. Drücke > 6 MPa, notwendig waren.

Sterisch anspruchsvolle Phosphite wie Tris(4-*tert*-butyl-2-methylphenyl)phosphit wurden von van Leeuwen und Mitarbeitern in die Hydroformylierung von Ölsäuremethylester getestet, wie in J. Am. Oil Chem. Soc. 1997, 74, 223 - 228 offenbart wird. Unter milden Bedingungen (2,0 MPa, CO : H₂ = 1:1, 80°C) wurde der Aldehyd mit 95% Ausbeute erhalten, vorausgesetzt der reine Ölsäuremethylester wurde eingesetzt. In Unterschied dazu war mit einem technischen Ölsäuremethylester (14% Linolsäuremethylester) die Ausbeute an 9/10-Formylprodukt sehr gering.

Andere Untersuchungen zu diesem Sachgebiet wurden von da Rosa und Gregório publiziert. In diesen Arbeiten wurde die Hydroformylierung von Ölsäuremethylester mit unterschiedlichen Rhodiumkomplexen, wie ([RhH(CO)(PPh₃)₃], [RhCl₃x3H₂O], [RhCl(CO)(PPh₃)₃], [Rh(OMe)(COD)]₂ bzw. [Rh(CO)₂(acac)]) und verschiedenen Liganden (Triphenylphosphin und Triphenylphosphit) erforscht. Bis 90-95% Ausbeute an Aldehyd wurden bei 100°C, 4,0 MPa (CO : H₂ = 2:1) erzielt, wie in J. Braz. Chem. Soc. 2005, 16, 1124 - 1129 und Quim. Nova 2012, 35, 1940 - 1944 offenbart wird.

Aufgabe der vorliegenden Erfindung ist es Liganden bereitzustellen, welche in der Hydroformyliuerung von Ölsäureester-haltigen Gemischen die zuvor im Stand der Technik beschriebenen Nachteile nicht aufweisen. Dies speziell vor dem Hintergrund einer isomerisierungsarmen Hydroformylierung und eines hohen Umsatzes an Ölsäurestern zu den bevorzugt in C₈ - C₁₃-Position formylierten Derivaten.

Diese Aufgabe wird gelöst durch Liganden der allgemeinen Formeln (I) oder (II):

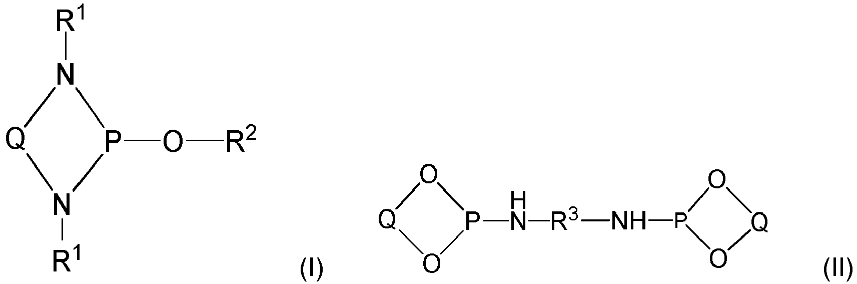

Ein Gegenstand der vorliegenden Erfindung sind Liganden der allgemeinen Formel (I) oder (II),
wobei Q für einen substitutierten oder unsubstituierten aromatischen Rest steht; wobei R¹ ausgewählt ist aus Alkyl-, Aryl-, Organosulfonylresten;
wobei R² ausgewählt ist aus einem substitutierten oder unsubstituierten aromatischen Rest;
wobei R³ für einen substitutierten oder unsubstituierten Alkylrest steht.

In einer besonderen Ausführungsform ist Q ausgewählt ist aus substituierten oder unsubstituierten 1,1'-Biphenyl- oder 1,1'-Binaphthylresten.

In einer Variante dieser Ausführungsform ist R¹ ausgewählt aus einem C₁ - C₄ - Alkyl-, einem Alkylsulfonyl-, einem Arylsulfonylrest, ist R² ein mindestens einfach substituierter aromatischer Rest, ist R³ ein substitutierter oder unsubstituierter Alkylrest mit mindestens 3 C-Atomen.

In einer besonders bevorzugten Ausführungsform sind die Liganden der Formeln (I), (II) ausgewählt unter **L9 - L14:**

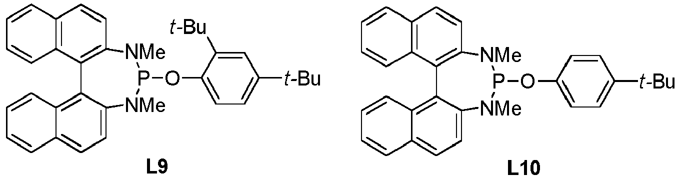

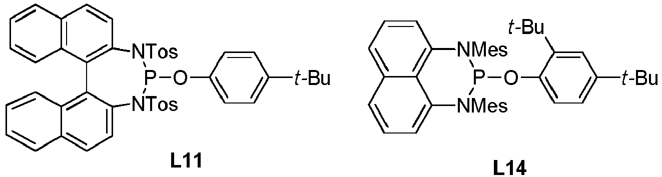

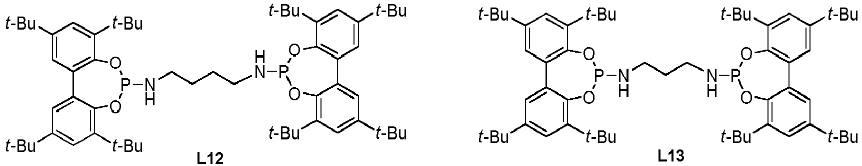

Ein weiterer Gegenstand der vorliegenden Erfindung sind übergangsmetallhaltige Verbindungen der Formel Me(acac)(CO)L mit Me = Übergangsmetall der 9.Gruppe des Periodensystems der Elemente, mit acac = Enolat-Anion des Acetylacetons, wobei L = Ligand ausgewählt ist unter: 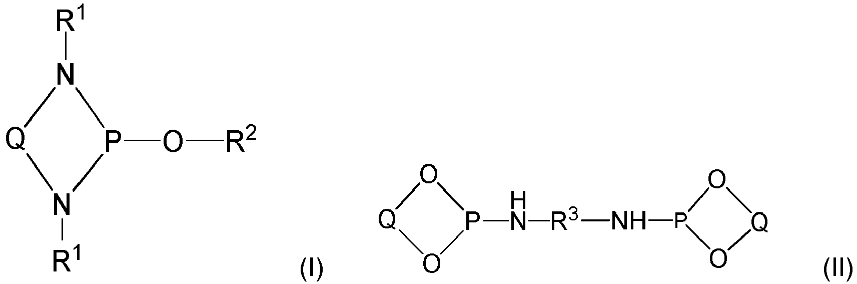 wobei Q für einen zweibindigen substitutierten oder unsubstituierten aromatischen Rest steht;
wobei R¹ ausgewählt ist aus Alkyl-, Aryl-, Organosulfonylresten;
wobei R² ausgewählt ist aus einem substitutierten oder unsubstituierten aromatischen Rest;
wobei R³ für einen substitutierten oder unsubstituierten Alkylrest steht.

In einer besonderen Ausführungsform ist Q ausgewählt ist aus substituierten oder unsubstituierten 1,1'-Biphenyl- oder 1,1'-Binaphthylresten.

In einer Variante dieser Ausführungsform ist R¹ ausgewählt aus einem C₁ - C₄ - Alkyl-, einem Alkylsulfonyl-, einem Arylsulfonylrest, ist R² ein mindestens einfach substituierter aromatischer Rest, ist R³ ein substitutierter oder unsubstituierter Alkylrest mit mindestens 3 C-Atomen.

In einer besonders bevorzugten Ausführungsform ist die Verbindung der Formel Me(acac)(CO)L mit Me = Übergangsmetall der 9. Gruppe des Periodensystems der Elemente, mit acac = Enolat-Anion des Acetylacetons,wobei L = Ligand ausgewählt ist unter:

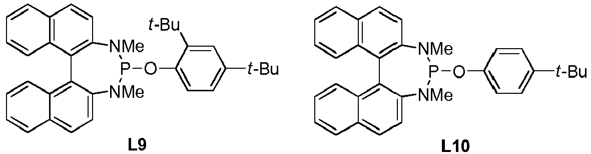

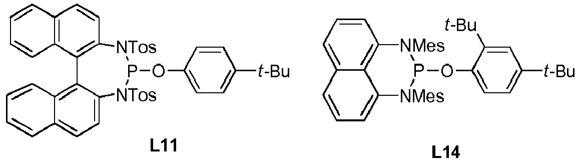

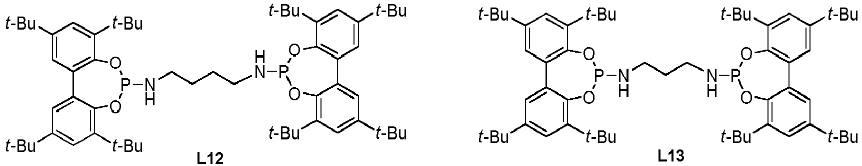

In einer Variante dieser Ausführungsform, in der das Übergangsmetall Rhodium ist, liegt das Ligand-Rhodium-Verhältnis im Bereich von 100:1 bis 1:1. Vorzugsweise liegt das Ligand-Rhodium-Verhältnis im Bereich von 50:1 bis 5:1, besonders bevorzugt im Bereich von 30:1 bis 20:1.

Das Übergangsmetall wird als Vorstufe in Form seiner Salze, wie beispielsweise den Halogeniden, Carboxylaten - z.B. Acetaten - oder kommerziell erhältlicher Komplexverbindungen, wie z. B. Acetylacetonaten, Carbonylen, Cyclopolyenen - z.B. 1,5-Cyclooctadien - oder auch deren Mischformen, wie z.B. Rh(acac)(CO)₂, Rh(acac)(COD) mit COD = 1,5-Cycloocatdien, mit den erfindungsgemäßen Verbindungen der Formeln **L9 - L14** in Kontakt gebracht, wobei diese Umsetzung in einer vorgelagerten Reaktion als auch in Gegenwart eines Wasserstoff- und Kohlenmonoxidhaltigen Gasgemisches erfolgen kann.

Mit ein Gegenstand der vorliegenden Erfindung sind Zusammensetzungen enthaltend:
a) Ölsäureester-haltiges Gemisch;
b) optional mindestens ein Lösungsmittel;
c) mindestens ein Ligand der Formeln (I) oder (II)
d) CO- und H₂-haltiges Synthesegasgemisch;
e) mindestens ein Übergangsmetall aus der 9. Gruppe des Periodensystems der Elemente.

In einer Ausführungsform weist die erfindungsgemäße Zusammensetzung neben dem Ölsäureester auch Linol-, Linolensäureester auf.

In einer Variante dieser Ausführungsform liegen die Ester in Form der Methylester vor.

In einer Variante dieser Ausführungsform werden die Liganden **L1 - L8** verwendet:

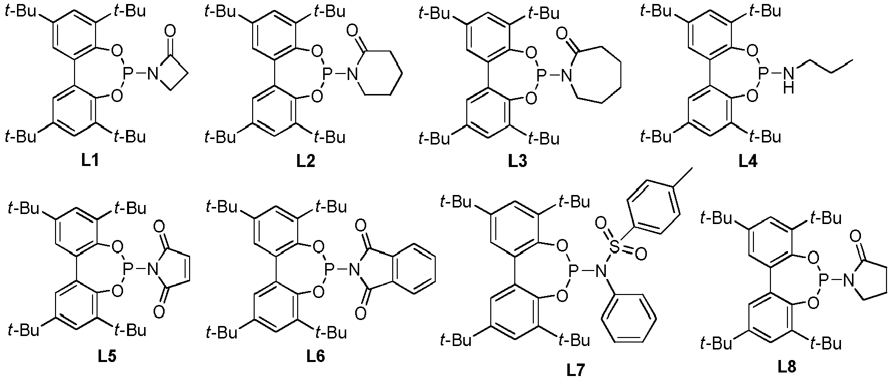

In einer Variante dieser Ausführungsform werden die Liganden **L9 - L14** verwendet.

In einer Ausführungsform der erfindungsgemäßen Zusammensetzung ist das Übergangsmetall Rhodium.

In einer weiteren Ausführungsform der erfindungsgemäßen Zusammensetzung ist das Lösungsmittel ausgewählt aus der Gruppe enthaltend aromatische Kohlenwasserstoffe, Alkohole, Ether, Carbonate.

In einer Variante dieser Ausführungsform weist der aromatische Kohlenwasserstoff Toluol oder Xylol, der Alkohol Methanol oder Ethanol, der Ether Tetrahydrofuran oder MTBE, das Carbonat Propylencarbonat auf.

Ein weiterer Gegenstand der vorliegenden Erfindung ist die Verwendung der erfindungsgemäßen Zusammensetzungen in einem Verfahren zur Hydroformylierung von Ölsäureester-haltigen Gemischen.

Das erfindungsgemäße Verfahren zur Hydroformylierung von Ölsäureester-haltigen Gemischen unter Verwendung der erfindungsgemäßen Zusammensetzungen weist Synthesegasdrücke in einem Bereich von 1 - 6 MPa und Reaktionstemperaturen in einem Bereich von 60 °C - 120 °C auf.

In einer Ausführungsform des erfindungsgemäßen Verfahrens liegt das Verhältnis von Ölsäureester-haltigem Gemisch zu Übergangsmetall in einem Bereich von 180 : 1 bis 910 : 1, das Verhältnis von 3-wertiger organischer Phosphorverbindung zu Übergangsmetall in einem Bereich von 1 : 1 bis 25 : 1.

In einer Variante dieser Ausführungsform werden als Liganden die folgenden Liganden **L1 - L8** verwendet:

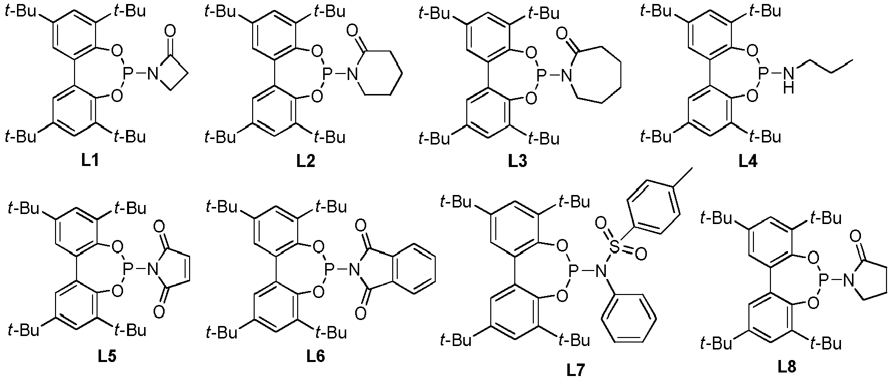

In einer Variante dieser Ausführungsform werden als Liganden die Liganden **L9 - L14** verwendet.

Die erfindungsgemäßen Zusammensetzungen sowie deren Verwendung werden nachfolgend beispielhaft beschrieben, ohne dass die Erfindung auf diese beispielhaften Ausführungsformen beschränkt sein soll. Sind nachfolgende Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Mit dem Begriff "inert" wird im Sinne der vorliegenden Erfindung die Eigenschaft von Stoffen, Komponenten oder Gemischen verstanden, welche sich dadurch auszeichnet, dass sich keine nachteiligen Auswirkungen oder dem beabsichtigten Reaktionsablauf gegenläufige Effekte einstellen.

### Beispiele

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen werden in ppm angegeben. Die Referenzierung der ³¹P-NMR-Signale erfolgte gemäß: SR_{31P} = SR_{1H} *(BF_{31P} / BF_{1H}) = SR_{1H} *0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Reaktionsschema: Hydroformylierung von Ölsäuremethylester.

Unter Hydroformylierungsbedingungen kann die Reaktion mit Ölsäuremethylester (MO) und seinen Gemischen als Substrat neben dem gewünschten C8-C13-Formylsteransäuremethylester (MFS) auch das isomerisierte Olefin (Elaidinsäuremethylester = ME) und das Hydrierprodukt (Stearinsäuremethylester = MS) liefern.

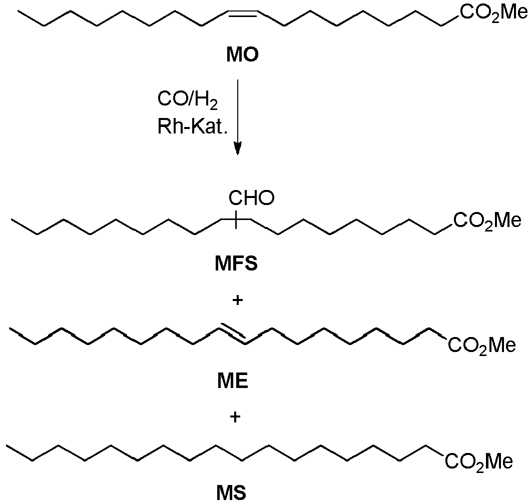

Vergleichsbeispiel: Isomerisierungsarme Hydrofomylierung von Ölsäuremethylester [Rh(acac)(CO)₂] (2,1 mg, 4,0 µmol) wurde unter Argon in einem Schlenkgefäß eingewogen und in 2 mL Toluol gelöst. 1 mL von dieser Lösung mit Ölsäuremethylester (2,0 mmol, 0,593 g, >99 % Reinheit), Triphenylphosphin (5,3 µmol), und 9 mL Toluol wurden in einen 25-mL Autoklav gefüllt. Der Autoklav wurde dreimal mit Stickstoff (1,0 MPa), einmal mit Synthesegas (CO:H2=1:1, 1,0 MPa) sekuriert und dann auf 80°C geheizt und der Druck auf 5,0 MPa eingestellt. Nach 24 h Reaktionszeit wird der Autoklav abgekühlt. Anschließend wurde bei Raumtemperatur der Druck abgelassen und zweimal mit Stickstoff gespült. Aus dem Autoklaven wurde eine Probe für die GC/MS Analyse entnommen. Das Lösungsmittel wurde evaporiert und das gelbe Öl per NMR (¹H, ¹³C, DEPT) analysiert; (99% Umsatz an MO, 99% MFS).

### Erfindungsgemäße Beispiele: Hydrofomylierung von Ölsäuremethylester mit den Liganden L1 - L14

[Rh(acac)(CO)₂] (1,4 mg, 5,43 µmol) wurde unter Argon in einem Schlenkgefäß eingewogen und in 5 mL Toluol gelöst. 1 mL von dieser Lösung mit Ölsäuremethylester (1,0 mmol, 0,296 g, >99 % Reinheit), Ligand (27,5 µmol), Octadecan (0,1 g) als interner Standard und 9 mL Toluol wurden in einen 25-mL Autoklav gefüllt. Der Autoklav wurde dreimal mit Stickstoff (1,0 MPa), einmal mit Synthesegas (CO:H2=1:1, 1,0 MPa) sekuriert und dann auf 80°C geheizt und der Druck auf 20 bar eingestellt. Nach 6 h Reaktionszeit wurde der Autoklav abgekühlt. Bei Raumtemperatur wurde der Druck abgelassen und zweimal mit Stickstoff nachgespült. Aus dem Autoklaven wurde eine Probe für die GC/MS Analyse entnommen. Das Lösungsmittel wurde evaporiert und das gelbe Öl per NMR (1 H, 13C, DEPT) analysiert.

### Analytik zur Bestimmung der Regioselektivität

Zur Charakterisierung und Kalibrierung des Produktes (MFS) wurde eine isomerisierungsfreie Hydroformylierung mit Triphenylphosphin entsprechend der Methode von Vogl et al., PhD Thesis, Rostock 2009, wie nachfolgend dargestellt, verwendet.

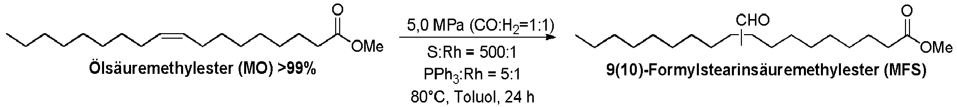

Zur Reinigung der Hydroformylierungsprodukte des Ölsäuremethylesters wurde die Reaktionsmischung in einer Kugelrohr-Destillationsapparatur destilliert (1.5x10⁻¹ mbar/180°C). Der reine Formylstearinsäuremethylester wurde für die Kalibrierung verwendet, wobei Octadecan als interner Standard eingesetzt wurde.

Bei Reinigungsversuchen des Produktes mittels Säulenchromatographie (Cyclohexan: Ethylacetat) zersetzte sich das Formylprodukt auf der Säule.

Um die genaue Position der Aldehydgruppe zu bestimmen, wurden die Produkte der Hydroformylierung durch GC/MS analysiert. Dass die Aldehyde sehr luftempfindlich sind, ist aus dem Stand der Technik bekannt; Frankel et al. in J. Am. Oil Chem. Soc. 1969, 46, 133 - 138 und an gleichem Ort 1971, 48, 248 - 253. Die Aldehyde wurden zu den entsprechenden Säuren oxidiert und dann in die Methylester überführt. Letztere wurden dann durch GC/MS analysiert. Das nachfolgende Schema fasst die Schritte zusammen.

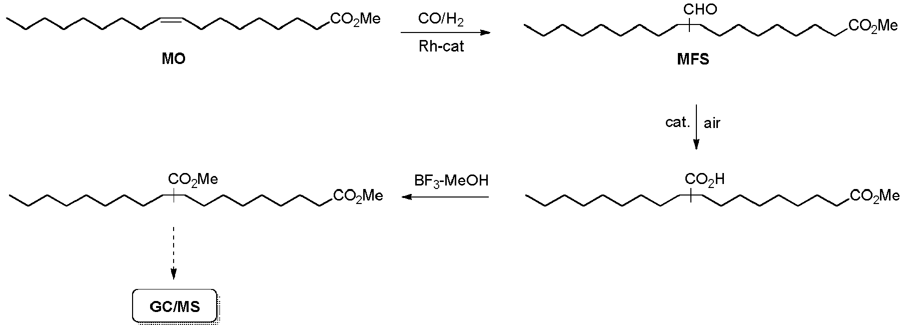

Um die genaue Position der Aldehydgruppe zu bestimmen, wurden die Produkte der Hydroformylierung in die entsprechenden Methylester umgewandelt und mittels GC/MS analysiert. Die verzweigten Formylprodukte, die aus der Hydroformylierung zwischen den Kohlenstoffatomen 3 bis 17 stammen, sind durch das Fragment CH3(CH2)ₙCHC(O+·H)OCH3 und für das lineare Produkt (18-MFS) durch das Fragment CHC(O+·H)OCH3 charakterisiert.

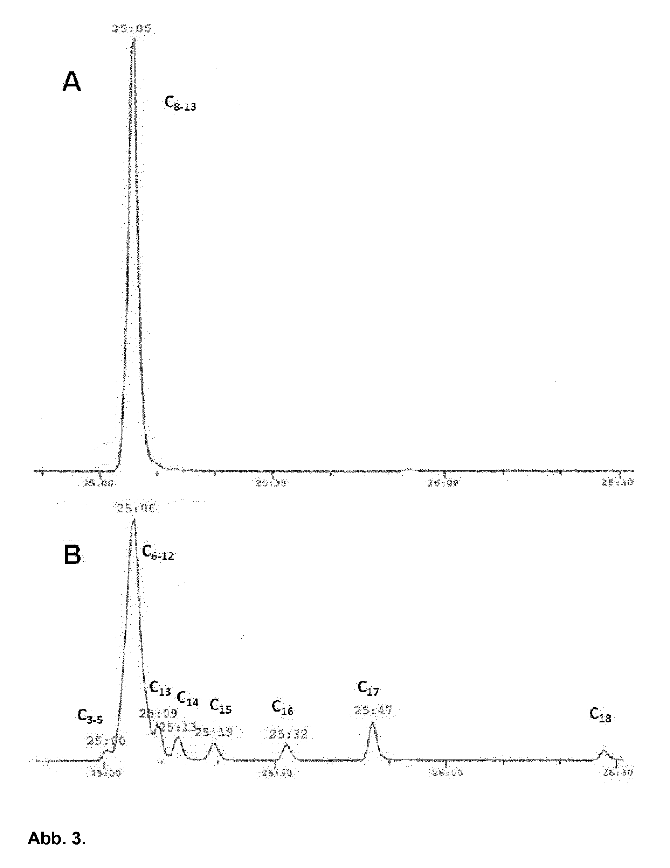

Das gaschromatographisch aufgelöste Massenspektrum A in Abbildung 3, das aus der Hydroformylierung bei 20 bar und 80 °C resultiert, beobachtet man einen einzelnen großen Peak, der hauptsächlich die Mischung von 9- (Fragment 200 m/z) und 10-MFS (Fragment 186 m/z) charakterisiert. Daneben wird eine kleinere Menge von 8- (214 m/z), 11- (172 m/z), 12- (158 m/z) und 13-MFS (144 m/z) gefunden. Diese Regioisomere befinden sich am Anfang und Ende des Gaschromatogramms; eine Trennung von den Hauptisomeren gelingt nicht.

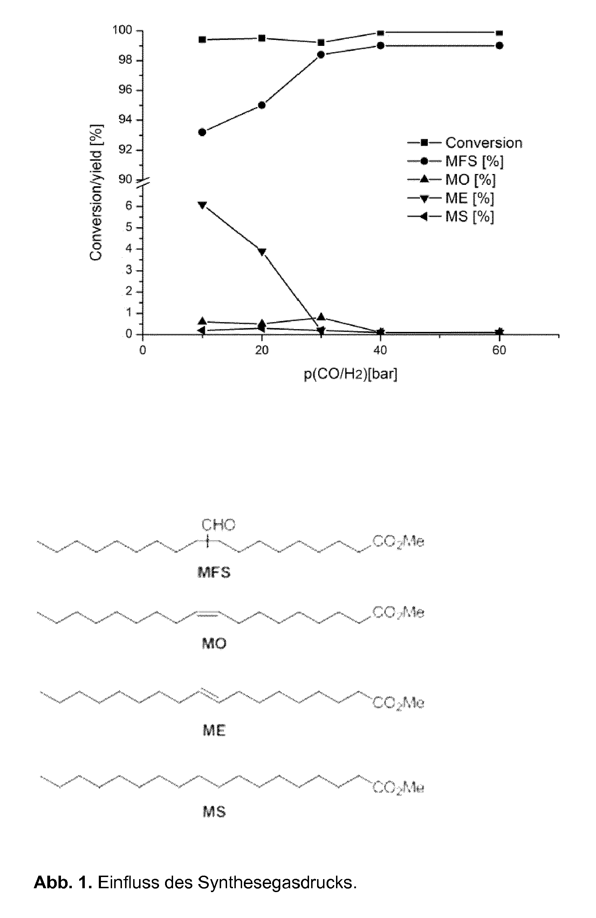

Das gaschromatographisch aufgelöste Massenspektrum B in Abbildung 3, welches nach der Aufarbeitung der Hydroformylierung bei 20 bar bei 120 °C aufgenommen wurde, zeigt einen geringeren Grad an Regioselektivität an, was durch das Auftreten zusätzlicher Peaks bewiesen wird. Der erste einzelne Peak entspricht der Mischung von 3-(284 m/z), 4-(270 m/z) und 5-MFS (256 m/z). Der Hauptpeak charakterisiert, wie bereits in Abbildung 1A gezeigt, eine Mischung aus 6- bis 12-MFS (242 m/z und 228 m/z für 6- und 7-MFS). Die Produkte 13-MFS (144 m/z), 14- (130 m/z), 15- (116 m/z), 16- (102 m/z), 17- (88 m/z), und 18-MFS (74 m/z) werden gut getrennt und können somit als einzelnen Peaks beobachtet werden.

### Massenspektrum der isomeren Methyl 13- bis 18-methoxycarboxystearate:

Methyl 13-methoxycarboxystearat: MS (EI, 70 eV): m/z: 325, 297, 254, 222, 144, 98, 87,55,41.

Methyl 14-methoxycarboxystearat: MS (EI, 70 eV): m/z: 324, 297, 268, 227, 154, 130, 98,87,55,41.

Methyl 15-methoxycarboxystearat: MS (EI, 70 eV): m/z: 325, 297, 241, 196, 140, 116, 98,87,55,41.

Methyl 16-methoxycarboxystearat: MS (EI, 70 eV): m/z: 324, 297, 255, 223, 182, 126, 102,87,55,41.

Methyl 17-methoxycarboxystearat: MS (EI, 70 eV): m/z: 324, 297, 269, 237, 210, 112, 98,88,55,41.

Methyl 18-methoxycarboxystearate: MS (EI, 70 eV): m/z : 325, 283, 251, 210, 112, 98, 74,55,41.

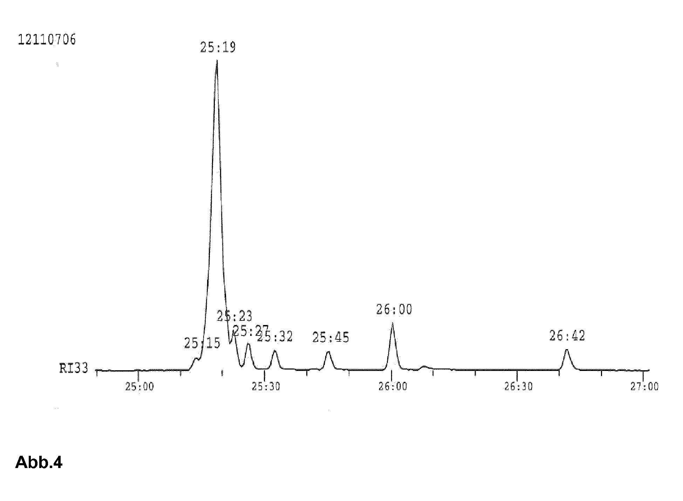

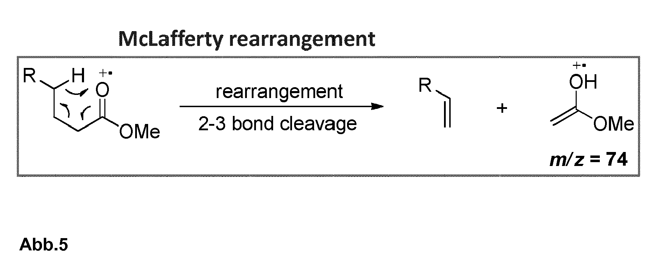

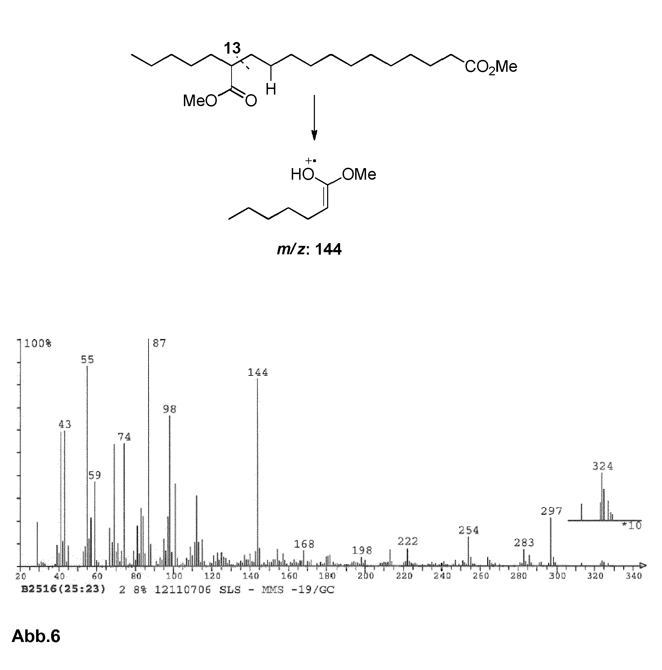

Die Zuordnung der jeweiligen Massenspektren und die daraus abgeleitete Quantifizierung an den jeweils hergestellten Formylstearat-Derivaten erfolgte auf Basis der dem Fachmann bekannten McLafferty-Umlagerung. Die Resultate sind in den Abbildungen 4, 5 und 6 offenbart.

### Mischung 9- und 10-Formyloctadecansäuremethylester.

1 H NMR (300 MHz, CDCl3): δ 0.91 (t, 3H, J = 7.0 Hz, CH3), 1.25-1.38 (m, 22H, CH2), 1.40-1.55 (m, 2H, CH2CH2CO2CH3), 1.57-1.73 (m, 3H, CH2CH(CHO)CH2), 2.20-2.30 (m, 1 H, CHCHO), 2.34 (t, 2H, J = 7.6 Hz, CH2CO2CH3), 3.70 (s, 3H, CO2CH3), 9.59 (d, 1 H, J = 3.2 Hz, CHO).

13C NMR (75 MHz, CDCl3): δ 14.1 (s, CH3), 22.7-32.0 (m, CH2), 34.1 (s, CH2CO2CH3), 34.1 (s, CH2CO2CH3), 51.5 (s, CO2CH3), 52.0 (s, CHCHO), 174.3 (s, C02CH3), 174.3 (s, CO2CH3), 205.7 (s, CHO), 205.7 (s, CHO).

MS (EI, 70 eV): m/z (I, %): 326 (<0.83), 298 (46.2), 269 (5.3), 255 (28.3), 241 (4.9), 213 (6.4), 199 (17.6), 171 (27.1), 139 (34.2), 125 (32.1), 111 (22.9), 98 (47.01), 87 (95.9), 74 (77.1), 69 (58.2), 55 (100), 43 (75.7).

### Oxidierung von Formyloctadecansäuremethylester

Ca. 100 mg Rohprodukt wurden 48 Stunden unter Luft stehen gelassen. Der erhaltene weiße Feststoff wurde per NMR (1 H, 13C, DEPT) analysiert; quantitative Umsetzung, 99% Ausbeute.

1 H NMR (300 MHz, CDCl3): δ 0,87 (t, 3H, J = 6,8 Hz, CH3), 1,15-1,37 (m, 22H, CH2), 1,39-1,51 (m, 2H, CH2), 1,53-1,71 (m, 4H, CH2), 2,29 (t, 2H, J = 7.6 Hz, CH2CO2CH3), 2,35 (m, 1 H, CHCO2H), 3,66 (s, 3H, CO2CH3).

13C NMR (75 MHz, CDCl3): δ 14,3 (s, CH3), 22,8-32,3 (m, CH2), 34,2 (s, CH2CO2CH3), 34,2 (s, CH2CO2CH3), 45,6 (s, CHCO2H), 51,6 (s, CO2CH3), 174.5 (s, CO2CH3), 174.5 (s, CO2CH3), 182.4 (s, CO2H).

### Veresterung des Carboxyloctadecansäuremethylester

Ca. 30 mg der freien Säure wurden in einem Reagenzglas in 1,0 mL Toluol gelöst. Anschließend wurden 2,0 mL einer Lösung aus Bortrifluorid in Methanol (10%) und drei Tropfen 2,2-Dimethoxypropan als wasserentziehendes Agens zuzugegeben. Die Reaktionsmischung wurde 25 Minuten auf 60 °C in einem Wasserbad gewärmt. Nach der Zugabe von 1,0 mL dest. Wasser wurden die Phasen getrennt. Die organische Phase wurde mit Na2SO4 getrocknet und dann filtriert; quantitative Umsetzung, 99% Ausbeute. Die Lösung wurde durch GC/MS analysiert.

MS (EI, 70 eV): m/z (I, %): 324 (<1.40), 297 (28.9), 283 (8.0), 264 (6.3), 244 (8.6), 230 (10.7), 212 (25.4), 200 (46.7), 186 (45.7), 157 (25.1), 143 (35.94), 112 (20.2), 98 (93.7), 87 (100), 74 (42.3), 69 (42.6), 55 (80.3), 43 (47.7).

### Verwendung von L1 als Ligand

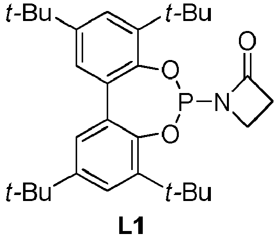

### Einfluss des Synthesegasdrucks

Die Hydroformylierung von MO wurde bei einer Reaktionstemperatur von 80 °C und fünf verschiedene Drücken (1,0, 2,0, 3,0, 4,0 und 6,0 MPa Synthesegas CO:H₂=1:1) getestet (Abbildung 1).

**Tabelle 1: Hydroformylierung von MO bei unterschiedlichen Drücken mit L1 als Ligand.^{a}**

| ***p* [MPa]** | **Umsatz [%]^{b}** | **MO [%]^{c}** | **MFS [%]^{c}** | **ME [%]^{c}** | **MS [%]**^{c} | **Regioselekivität^{d}** |
|---|---|---|---|---|---|---|
| 1,0 | 99,4 | 0,6 | 93,2 | 6,1 | 0,2 | C5-C17 |
| 2,0 | 99,5 | 0,5 | 95,0 | 3,9 | 0,3 | C8-C13 |
| 3,0 | 99,2 | 0,8 | 98,4 | 0,2 | 0,2 | C8-C13 |
| 4,0 | 99,9 | - | 99,0 - | | - | C8-C13 |
| 6,0 | 99,9 | - | 99,0 - | | - | C8-C12 |

| | | | | | | |
|---|---|---|---|---|---|---|
| ^{a}Reaktionsbedingungen: 1 mmol Substrat (S), S:Rh = 910:1, P:Rh = 25:1, 80 °C, 10 mL Toluol. ^{b}Umsätze wurden durch GC bestimmt (100 - % GC Ausbeute von MO). ^{c}Durch GC bestimmt. ^{d}Cn, n = Kohlenstoffatome, wo die Hydroformylierung stattfand. | | | | | | |

In Tabelle 1 wird deutlich, dass eine Druckerhöhung zu höherer Ausbeute an MFS führt, aber in diesem Fall ist die Steigerung ab 2,0 MPa sehr stark (von 95% bei 2,0 MPa zu >98% bei 3,0 MPa). Bei höheren Gasdrücken (> 2,0 MPa) verbessert sich die Chemoselektivität in Richtung Hydroformylierung. In allen Fällen war die Bildung des Hydrierproduktes MS sehr niedrig (0,1-0,3%).

Die Regioselektivität ist ebenfalls abhängig von Synthesegasdruck. Bei höheren Drücken nimmt die Regioselektivität zu (Tabelle 1).

### Einfluss der Reaktionstemperatur

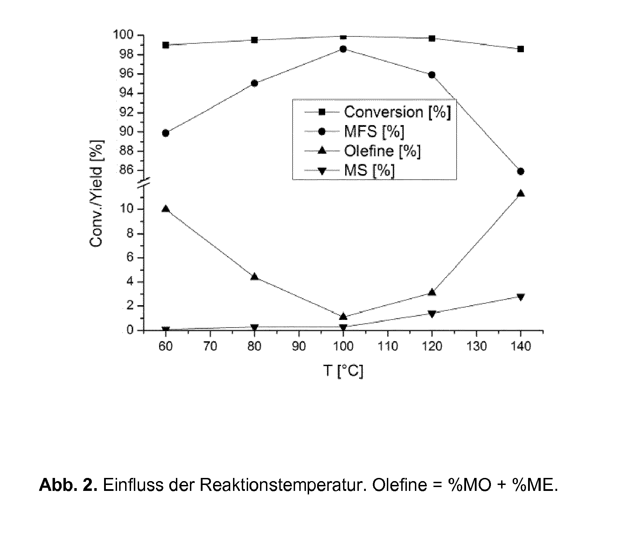

Die Reaktion wurde bei 2,0 MPa bei fünf verschiedenen Temperaturen (60, 80, 100, 120, 140 °C) durchgeführt (Abbildung 2).

**Tabelle 2: Hydroformylierung von MO bei unterschiedlichen Temperaturen mit L1 als Ligand.^{a}**

| **T [°C]** | **Umsatz [%]^{b}** | **MO [%]^{c}** | **MFS [%]^{c}** | **ME [%]^{c}** | **MS [%]^{c}** | **Regioselectivität^{d}** |
|---|---|---|---|---|---|---|
| 60 | 99,0 | 1,0 | 89,9 | 9,0 | 0,1 | C9-C12 |
| 80 | 99,5 | 0,5 | 95,0 | 3,9 | 0,3 | C8-C13 |
| 100 | 99,9 | 0,1 | 98,6 | 1,0 | 0,3 | C3-C18 |
| 120 | 99,7 | 0,3 | 95,9 | 2,8 | 1,4 | C3-C18 |
| 140 | 98,6 | 1,4 | 85,9 | 9,9 | 2,8 | C3-C18 |

| | | | | | | |
|---|---|---|---|---|---|---|
| ^{a}Reaktionsbedingungen: 1 mmol Substrat (S), S:Rh = 910:1, P:Rh = 25:1, 2,0 MPa Synthesegas (CO:H₂=1:1), 10 mL Toluol, ^{b}Umsätze wurden durch GC bestimmt (100 - % GC Ausbeute von MO). ^{c}Durch GC bestimmt. ^{d}Cn, n= Kohlenstoffatome, wo die Hydroformylierung stattfand. | | | | | | |

Die Chemoselektivität nahm auch bei höheren Temperaturen zunächst zu. Bei einem Maximum von 100°C nahm sie bei 120 und 140 °C wieder ab. Offenbar können bei Temperaturen höher als 120 °C drei Reaktionen miteinander konkurrieren: 1.) Hydroformylierung zum MFS 2.) Decarbonylierung zum entsprechende Olefin und 3.) Hydrierung.

Bei 100, 120 und 140 °C nimmt die Regioselektivität deutlich ab (Tabelle 2).

### Einfluss des Lösungsmittels

Wenn die Hydroformylierung von Ölsäuremethylester in Methanol als Lösungsmittel durchgeführt wurde, nehmen Umsatz (39,1%) und Ausbeute von MFS (25,7%) ab. Obwohl mit diesem Lösungsmittel die beste Regioselektivität (nur die Mischung C9-C10) gefunden wird, treten auch massenspektrokopisch nicht identifizierbare Produkte im Gaschromatogramm auf.

### Variation der Liganden

Unter optimierten Bedingungen (2,0 MPa, 80 °C, Toluol) wurden Phosphoramidite als Liganden in der Hydroformylierung von Ölsäuremethylester getestet.

Zum Vergleich wurden die Ergebnisse mit kommerziell erhältlichem Triphenylphosphin gegenübergestellt. 12 monodentate und 2 bidentate Liganden wurden untersucht:

**Tabelle 3: Hydroformylierung von MO mit erfindungsgemäßen Liganden L1-L14^{a} im Vergleich zu PPh₃**

| **Ligand** | **Umsatz [%]^{b}** | **MO [%]^{c}** | **MFS [%]^{c}** | **ME [%]^{c}** | **MS [%]^{c}** |
|---|---|---|---|---|---|
| **PPh₃** | 59,0 | 41,0 | 51,8 | 7,1 | 0,1 |
| **L1^{d}** | 99,5 | 0,5 | **95,0** | 3,9 | 0,3 |
| **L2** | 88,7 | 11,3 | 32,2 | 56,2 | 0,3 |
| **L3** | 98,7 | 1,3 | **89,1** | 9,3 | 0,3 |
| **L4** | 92,9 | 7,1 | 43,8 | 49,0 | 0,1 |
| **L5** | 46,2 | 53,8 | 0,7 | 45,2 | 0,3 |
| **L6** | 91,6 | 8,4 | 39,9 | 51,5 | 0,2 |
| **L7** | 95,8 | 4,2 | 54,7 | 28,9 | 1,1 |
| **L8** | 91,5 | 8,5 | 32,9 | 58,1 | 0,5 |
| **L9** | 32,8 | 67,2 | 25,5 | 7,3 | 0 |
| **L10** | 92,3 | 7,7 | 27,7 | 64,3 | 0,3 |
| **L11** | 94,4 | 5,6 | 39,5 | 54,3 | 0,6 |
| **L12** | 68,6 | 31,4 | 51,5 | 17,0 | 0,1 |
| **L13** | 1,6 | 98,3 | 1,5 | 0,1 | 0,1 |
| **L14** | 93,5 | 6,5 | 32,3 | 60,1 | 1,2 |
| **L4^{e}** | 91,4 | 8,6 | 38,2 | 52,8 | 0,4 |

| | | | | | |
|---|---|---|---|---|---|
| ^{a}Reaktionbedingungen: 1.0 mmol of Substrat (S), S:Rh = 910:1, P:Rh = 25:1, 20 bar Synthesegas (CO:H₂=1:1), 80 °C, 10 mL Toluol (Die Reaktionmischung wurde 6 h bei 80 °C gerührt und danach wurde die weitergerührt bei Raumtemperatur unter Syngas-Druck bis Bearbeitung). ^{b}Umsätze wurden durch GC bestimmt (100 - % GC Ausbeute von MO). ^{c}Durch GC bestimmt. ^{d}Durchschnitt zwischen zwei Reaktionen, ^{e}nach 6 h wurde die Reaktion aufgearbeitet und die Probe durch GC/MS analysiert. | | | | | |

Die besten Ergebnisse wurden für die 4-Ring und 7-Ring Lactame **L1** und **L3** mit einer Ausbeute von 92,9% und 89,1% an MFS erzielt. Der im Stand der Technik genannte und kommerziell verfügbare Ligand Triphenylphosphin zeichnet sich unter diesen im Vergleich zum Stand der Technik deutlich milderen Reaktionsbedingungen durch einen ungenügenden Umsatz von lediglich knapp 60% aus.

Als Fazit wird festgestellt, dass eine Hydroformylierung von Ölsäureester-haltigen Gemischen mit Umsätzen > 85 % zu den in C8 - C13-Position formylierten Derivaten unter Verwendung der Liganden **L1** und **L3** zugänglich ist. Die Hydroformylierung wird bevorzugt innenständig, somit isomerisierungsarm durchgeführt,

## Patentansprüche

1. Ligand der allgemeinen Formeln (I) oder (II) 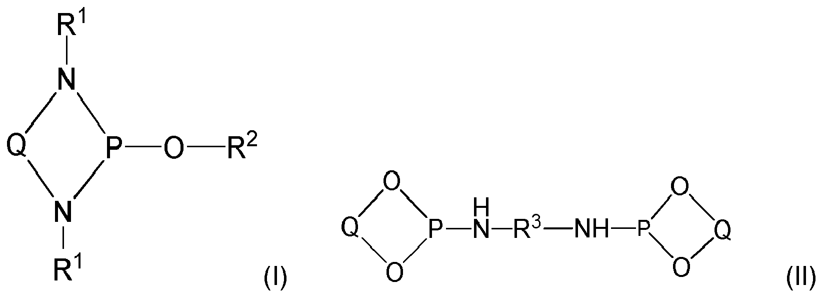 wobei Q für einen substitutierten oder unsubstituierten aromatischen Rest steht; wobei R¹ ausgewählt ist aus Alkyl-, Aryl-, Organosulfonylresten;
wobei R² ausgewählt ist aus einem substitutierten oder unsubstituierten aromatischen Rest;
wobei R³ für einen substitutierten oder unsubstituierten Alkylrest steht.

2. Ligand nach Anspruch 1,
wobei Q ausgewählt ist aus substituierten oder unsubstituierten 1,1'-Biphenyl- oder 1,1'-Binaphthylresten.

3. Ligand nach den Ansprüchen 1 oder 2, wobei
R¹ ausgewählt ist aus einem C₁ - C₄ - Alkyl-, einem Alkylsulfonyl-, einem Arylsulfonylrest,
R² ein mindestens einfach substituierter aromatischer Rest ist,
R³ ein substitutierter oder unsubstituierter Alkylrest mit mindestens 3C-Atomen ist.

4. Ligand nach Anspruch 3,
ausgewählt aus der Gruppe enthaltend:

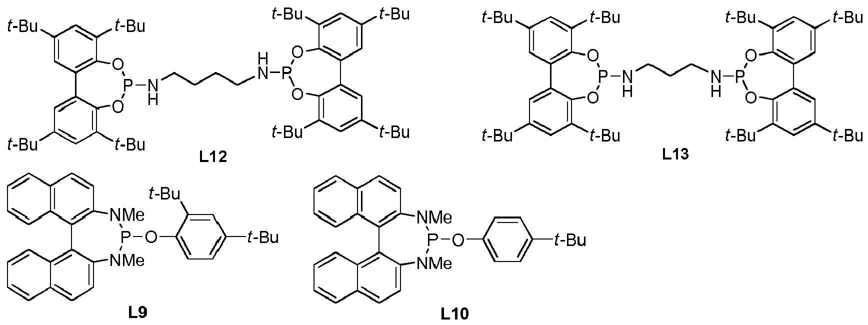

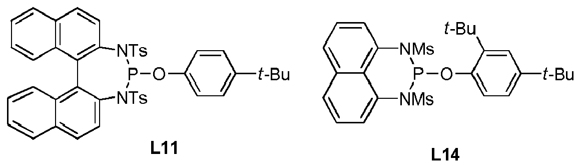

5. Übergangsmetallhaltige Verbindungen der Formel Me(acac)(CO)L mit Me = Über-gangsmetall der 9. Gruppe, wobei L = Ligand ausgewählt ist unter: 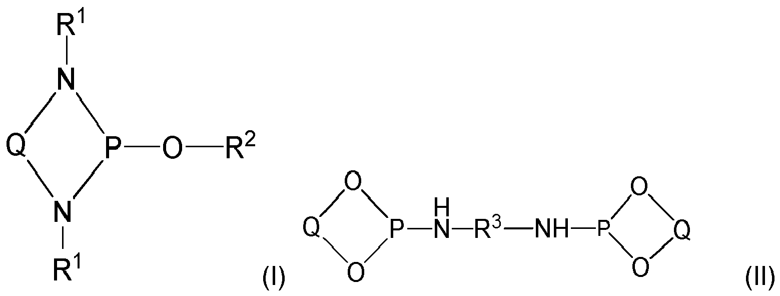 wobei Q für einen substitutierten oder unsubstituierten aromatischen Rest steht; wobei R¹ ausgewählt ist aus Alkyl-, Aryl-, Organosulfonylresten;
wobei R² ausgewählt ist aus einem substitutierten oder unsubstituierten aromatischen Rest;
wobei R³ für einen substitutierten oder unsubstituierten Alkylrest steht.

6. Übergangsmetallhaltige Verbindungen nach Anspruch 5,
wobei Q ausgewählt ist aus substituierten oder unsubstituierten1,1'-Biphenyl- oder 1,1 '-Binaphthylresten.

7. Übergangsmetallhaltige Verbindungen nach mindestens einem der Ansprüche 5 - 6, wobei
R¹ ausgewählt ist aus einem C₁ - C₄ - Alkyl-, einem Alkylsulfonyl-, einem Arylsulfonylrest,
R² ein mindestens einfach substituierter aromatischer Rest ist,
R³ ein substitutierter oder unsubstituierter Alkylrest mit mindestens 3 C-Atomen ist.

8. Übergangsmetallhaltige Verbindungen nach mindestens einem der Ansprüche 5 - 7, wobei L = Ligand ist ausgewählt unter:

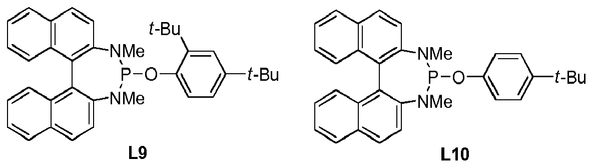

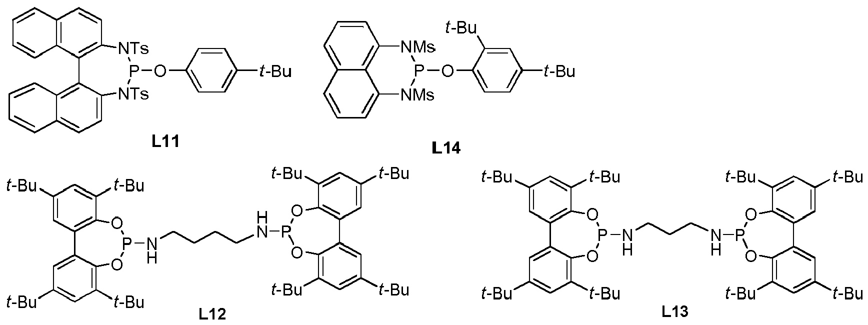

9. Übergangsmetallhaltige Verbindungen nach mindestens einem der Ansprüche 5 - 8, **dadurch gekennzeichnet, dass** sie Rhodium aufweist.

10. Zusammensetzung, enthaltend:
a) Ölsäureester-haltiges Gemisch;
b) optional mindestens ein Lösungsmittel;
c) mindestens eine Liganden nach einem der Ansprüche 1 bis 4 oder einen der Liganden L1 bis L8: 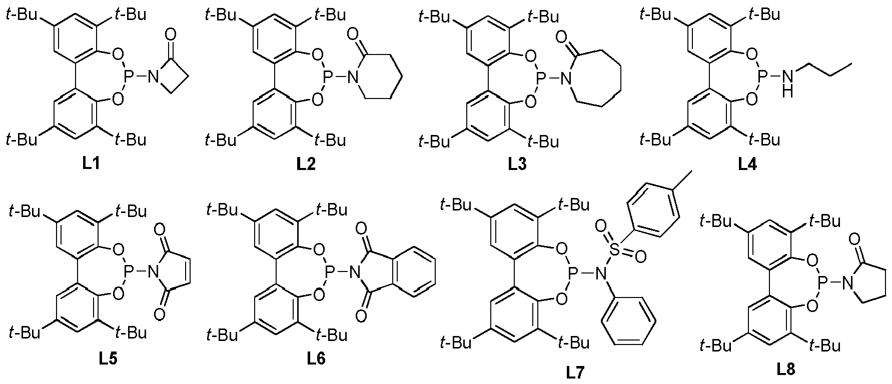
d) CO- und H₂-haltiges Synthesegasgemisch;
e) mindestens ein Übergangsmetall aus der 9. Gruppe des Periodensystems der Elemente.

11. Zusammensetzung nach 10,
wobei neben dem Ölsäureester auch Linol-, Linolensäurester anwesend sind.

12. Zusammensetzung nach 11,
wobei die Ester Methylester aufweisen.

13. Zusammensetzung nach 12,
die eine der folgenden Liganden aufweist:

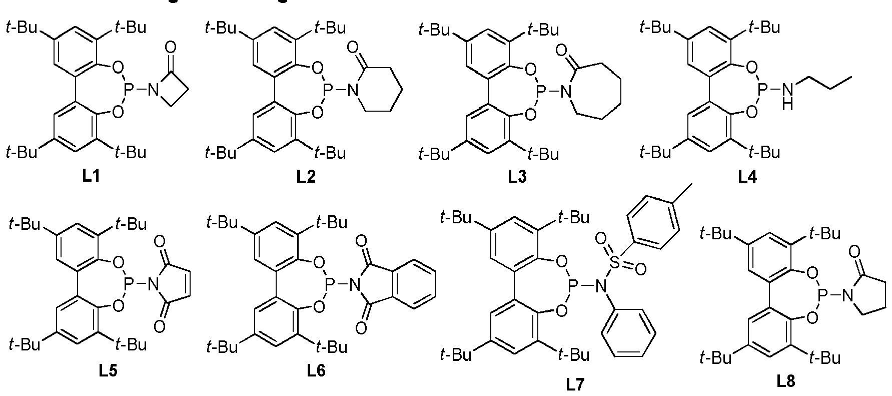

14. Zusammensetzung nach mindestens einem der Ansprüche 10 - 13, wobei das Übergangsmetall Rhodium ist.

15. Zusammensetzung nach mindestens einem der Ansprüche 10 - 14,
wobei das Lösungsmittel ausgewählt ist aus der Gruppe enthaltend aromatische Kohlenwasserstoffe, Alkohole, Ether, Carbonate.

16. Zusammensetzung nach 15,
wobei der aromatische Kohlenwasserstoff Toluol oder Xylol, der Alkohol Methanol oder Ethanol, der Ether Tetrahydrofuran oder MTBE, das Carbonat Propylencarbonat aufweist.

17. Verwendung einer Zusammensetzung nach mindestens einem der Ansprüche 10-16 in einem Verfahren zur Hydroformylierung von Ölsäureester-haltigen Gemischen.

18. Verfahren zur Hydroformylierung von Ölsäureester-haltigen Gemischen unter Verwendung einer Zusammensetzung nach einem der Ansprüche 10 - 16, wobei der Synthesegasdruck in einem Bereich von 1 - 6 MPa und die Reaktionstemperatur in einem Bereich von 60 °C - 120 °C liegt.

19. Verfahren nach 18, wobei das Verhältnis Ölsäureester-haltiges Gemisch zu Übergangsmetall in einem Bereich von 180 : 1 bis 910 : 1, das Verhältnis von 3-wertiger organischer Phosphorverbindung zu Übergangsmetall in einem Bereich von 1 : 1 bis 25 : 1 liegt.
